# EUROPÄISCHE PATENTSCHRIFT

(11) **EP 0 750 476 B1**
(45) Veröffentlichungstag und Bekanntmachung des Hinweises auf die Patenterteilung: **02.06.1999**
(21) Anmeldenummer: 95913127.7
(22) Anmeldetag: 15.03.1995
(51) Int. Cl.: A61B 17/36

(54) **LASERKATHETER ZUR BYPASS-CHIRURGIE**
LASER CATHETER FOR BYPASS SURGERY
CATHETER-LASER POUR CHIRURGIE DE PONTAGE

(30) Priorität: 15.03.1994 DE 4408746
(43) Veröffentlichungstag der Anmeldung: 02.01.1997
(73) Patentinhaber: MEDOLAS Gesellschaft für Medizintechnik, 92224 Amberg (DE)
(72) Erfinder: TULLEKEN, C., A., F., NL-1261 AH Blaricum (NL); VERDAASDONK, R., M., NL-3992 XA Houten (NL); VAN MANSVELT-BECK, H., J., NL-3721 XK Bilthoven (NL)
(74) Vertreter: Münich, Wilhelm, Dr.
(86) Internationale Anmeldenummer: EP9500968
(87) Internationale Veröffentlichungsnummer: WO9524869

(56) Entgegenhaltungen:
- EP-A- 0 141 536
- EP-A- 0 335 552
- WO-A-87/04611
- WO-A-88/04157
- WO-A-91/01690
- WO-A-91/05332
- US-A- 3 805 793

## Beschreibung

### Technisches Gebiet

Die Erfindung bezieht sich auf einen Laserkatheter zur gezielten Applikation von Laserlicht an intrakorporalen Gefäßwänden, mit ringförmig angeordneten Lichtleitfasern, die einen inneren Hohlkanal umschließen.

### Stand der Technik

Der Einsatz von Laserkathetern in der Chirurgie findet zunehmende Einsatzmöglichkeiten, die nicht zuletzt durch die fortschreitende Vielzahl unterschiedlicher konstruktiver Ausführungsformen von Laserkathetern und deren distale Endbereiche ermöglicht werden.

So sind Laserkatheterspitzen bekannt, die insbesondere für Bypass-Operationen verwendbar sind, die sich insbesondere dadurch auszeichnen, daß der Querschnitt des distalen Laserkatheterendes, an dem der Lichtaustritt erfolgt, vollständig aus Lichtleitfasern besteht. Diese Art von Katether werden bspw. als "Voll-Multifaser-Katether" bezeichnet.

Mit Hilfe des vorgenannten Kathetertyps sind Techniken für die laserunterstützte Anastomose (=chirurgische Verbindung zweier Hohlorgane, beispielsweise die Anbringung eines zusätzlichen blutführenden Kanals (Bypass) an eine blutführende Ader, deren Strömungsquerschnitt durch Ablagerungen verengt ist) bekannt, die bereits Bypass-Operationen ohne Unterbrechung der Blutströmung in der Hauptader gestatten. So ist beispielsweise aus den veröffentlichten Aufsätzen von Rudolf Verdaasdonk et al. ("End-to-Side anastomosis of small vessels using an Nd:YAG laser with a hemispherical contact probe", J. Neurosurg Vol. 76, March 1992; "Use of the excimer laser in high-flow bypass surgery of the brain", J. Neurosurg, Vol. 78, March 1993) eine Operationstechnik zu entnehmen, die mit Hilfe von Neodym-YAG- bzw. Excimer-Laser eine Bypass-Operation im Gehirnbereich vorstellen, ohne daß die von der Operation betreffende blutführende Hauptader in der Blutströmung beeinträchtigt werden muß.

So wird zunächst auf dem Außenumfang der zu operierenden Ader ein Bypass angenäht, durch den sodann eine im distalen Bereich elastisch ausgebildete Laserkatheterspitze einführbar ist und innerhalb des Bypasses auf die Außenwand der zu operierenden Hauptader positioniert wird. In dieser Anordnung wird der Laser aktiviert und erzeugt im Bereich des Lichtaustrittes am distalen Ende der Lichtleitfaser durch Ablationsprozesse ein Loch in der Hauptader, durch das sodann ein Teil der Blutströmung umgeleitet werden kann.

Neben dem großen Vorteil, Bypass-Operationen ohne Unterbrechung der Hauptader durchführen zu können, das insbesondere für die Bypass-Operationen im Gehirnbereich von essentieller Wichtigkeit ist, weist jedoch die vorgestellte Operationstechnik den Nachteil auf, daß das ablatierte Gefäßwandmaterial, das von der Lichtbeaufschlagung durch die Laserkatheterspitze herrührt, innerhalb der Blutbahn verbleibt und ungünstigenfalls an Blutbahnstellen kleineren Durchmessers gerät und diese vollständig blockiert. Besonders im Gehirnbereich sind deartige in den Blutbahnkreisläufen vorhandene Kleinstgewebeteilchen Ursache für einen spontan auftretenden Schlaganfall.

Ergänzend findet sich eine diesbezügliche Abhandlung über die beschriebene Bypass-Operation sowie die hierfür verwendeten Lichtleitfaseranordnungen für distale Endbereiche für Laserkathetern auch in einer Veröffentlichung des Medical Laser Center, Heart-Lung Institute, Department of Neurosurgery mit dem Titel "Multifiber excimer laser catheter design strategies for various medical applications" von R. Verdassdonk et al., Januar 1994.

### Darstellung der Erfindung

Der Erfindung liegt die Aufgabe zugrunde ein Laserkatheter zur gezielten Applikation von Laserlicht an intrakorporalen Gefäßwänden, mit ringförmig angeordneten Lichtleitfasern, die einen inneren Hohlkanal umschließen, derart auszugestalten, daß die Gefahr der Verstopfung blutführender Gefäße durch Ablationsreste nach einer Laserlichtapplikation vollständig auszuschließen ist und daß die Durchtrennung der zu behandelnden Gefäßwand exakt der Geometrie der Laserkatheterspitze entspricht, um einen vordefinierten Querschnitt für den Gefäßwanddurchbruch zu ermöglichen.

Die Lösung der, der Erfindung zugrunde liegenden Aufgabe ist im Anspruch 1 wiedergegeben.
Weitere, die Erfindungsgedanken vorteilhaft ergänzende Merkmale sind in den Unteransprüchen 2 bis 11 angegeben.

Der Erfindung liegt eine besondere Ausformung der Laserkatheterspitze zugrunde, mit der es erstmalig möglich ist, ein von der Geometrie exakt vordefiniertes Loch innerhalb einer zu behandelnden Gefäßwand zu erzeugen und gleichzeitig dafür zu sorgen, daß abgetrennte Gefäßwandreste nicht in der Blutbahn verbleiben, sondern mit Hilfe des Laserkatheters extrakorporal verbracht werden kann.

Hierfür weist der erfindungsgemäße Laserkatheter zur gezielten Applikation von Laserlicht an intrakorporalen Gefäßwänden, mit vollkreisringförmig angeordneten Lichtleitfasern, die einen inneren Hohlkanal umschließen, erfindungsgemäß am distalen Bereich des Laserkatheters an dessen Außenumfang ein den Außenumfang erweiterndes Element auf, durch das die Lichtleitfasern in Strahlrichtung hindurchtreten und als distale Laserkatheterspitze die Form einer ebenen, vollkreisringförmigen Lichtaustrittsfläche annehmen.

Das den Außenumfang des Laserkatheters erweiternde Element dient grundsätzlich als eine Art Anschlagvorrichtung, die es dem Operateur ermöglicht festzustellen, wann die maximale Durchdringungstiefe der am distalen Ende abstehenden, ringförmigen Lichtleitfasern durch den zu ablatierenden Gefäßwandbereich erreicht ist. Der Eindringvorgang ist vom Operateur spätestens dann zu beenden, wenn der Stoßrand des den Außenumfang erweiternden Elementes mit der Gefäßaußenwand selbst oder mit einem, mit der Gefäßaußenwand verbundenen Gegenstand in Kontakt tritt.

Durch die Kontaktierung des Stoßrandes mit der Gefäßaußenwand wird diese entsprechend der Ausformung des Stoßrandes ebenförmig gedrückt, so daß ein gleichmäßiger Aufsatz der Lichtleitfasern auf der Gefäßwand gewährleistet werden kann.

Ferner gestattet die vollkreisringförmige Anordnung der Lichtleitfasern das Abtrennen einer kreisförmigen Gefäßwand-scheibe, die im Inneren der Lichtleitfaseranordnung zu liegen kommt. Wird der Laserkatheter vorsichtig aus der durchtrennten Gefäßwand entnommen, so bleiben keine Ablationsrückstände im Blutkreislaufsystem zurück, da das kreisrund, abgetrennte Gefäßwandstück in vorteilhafter Weise durch einen im Inneren des Laserkatheters herrschenden Unterdruck in diesem gehalten wird.

Desweiteren ist der Laserkatheter vorzugsweise mit einem am distalen Bereich angeordneten perforierten Abschlußdeckel versehen, der den Hohlkanal distal abschließt. Die Beabstandung des Abschlußdeckels zur ebenen, vollkreisringförmigen Lichtaustrittsfläche der Lichtleitfaser ist derart bemessen, daß sie zumindest der Dicke der zu ablatierenden Gefäßwand entspricht. Eine derartige Dimensionierung bewirkt, daß die an sich elastische Gefäßwand durch den, in vorteilhafter Weise innerhalb des Hohlkanals herrschenden Unterdruck an die Oberseite des Abschlußdeckels flächenhaft gezogen wird, so daß zum einen die Lichtaustrittsfläche der Lichtleitfasern vollständig mit der umliegenden Gefäßwand in Berührung kommt und zum anderen die Gefäßwand an dem Innenprofil, das sich aus dem ringförmigen Lichtleitfaserkanal und dem Abschlußdeckel ergibt, weitgehend eng anliegt, um für eine kreisrunde Abtrennung der Gefäßwand zu garantieren.

Ferner ist erkannt worden, daß der Abtrennvorgang dadurch optimierbar ist, indem die Katheterspitze während der Laserlichtapplikation mit einem besonders angeordneten, ringförmig ausgebildeten Element in Wirkverbindung steht, so daß einerseits dafür gesorgt ist, daß die Abtrennung des Gefäßwandstückes vollständig erfolgt und eine weitere Gefäßwandverletzung, beispielsweise der gegenüberliegenden Gefäßwand der blutführenden Ader, auszuschließen ist. Das ringförmige Element ist getrennt von der Katetheterspitze mit dem Bypass-Kanal derart verbunden, daß es für eine Straffung der Gefäßwand während der Laserlichtapplikation sorgt, so daß die ringförmig ausgebildete Laserkatheterspitze gleichmäßig auf der Gefäßwand aufsitzt und Voraussetzung für eine homogene Lichtbeaufschlagung ist.

Die vorstehend genannte Laserkatheterspitze ermöglicht es erstmals, von außen an einer Gefäßwand einen kontrollierten Durchbruch durch die Gefäßwand zu erreichen, ohne dabei etwaige Abbrandprodukte, die während der Laserlichtapplikation an der Gefäßwand entstehen könnten, oder weitere Gefäßwandstückchen, die sich aus sonstigen Gründen während der Durchtrennung der Gefäßwand bilden könnten, zu erhalten. Somit ist das Gefahrenpotential einer durch Abbrandprodukte entstehenden Verstopfung an entfernter liegenden Engpässen in der Blutbahn, vollständig ausgeschlossen.

Als besonders vorteilhaft hat es sich erwiesen, daß der Hohlkanal, der von dem ringförmig ausgebildeten Lichtleitfasern umschlossen ist und an einer Unterdruckquelle angeschlossen ist, am distalen Ende mit einem Abschlußdeckel vorgesehen ist, der im Deckel konzentrisch angeordnete Bohrungen aufweist, die den Deckel derart perforieren, so daß eine gleichmäßige Ansaugwirkung erzielt werden kann, um die abgetrennte Gefäßwand gleichmäßig an der Deckeloberfläche durch den Unterdruck zu fixieren.

### Kurze Beschreibung der Zeichnung

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1: eine erfindungsgemäße Ausführungsform der Laserkatheterspitze,
- Fig. 2a, b, c, d: Darstellung der vorbereitenden Maßnahmen zur Durchführung einer Bypass-Operation mit der erfindungsgemäßen Laserkatheterspitze,
- Fig. 3: schematische Darstellung der Anbringung des Bypasses an einer zu behandelnden Gefäßwand,
- Fig. 4a, b, c: Darstellung der Funktionsweise der erfindungsgemäßen Laserkatheterspitze während der Lichtapplikation, und
- Fig. 5a, b: Darstellung der Entnahme der abgetrennten Gefäßwand mittels Laserkatheter.

### Beschreibung eines Ausführungsbeispiels

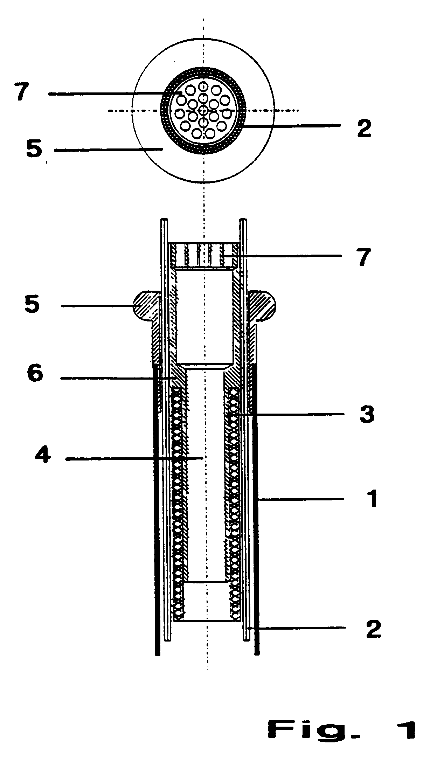

In Figur 1 ist eine Querschnittsdarstellung des distalen Bereiches des erfindungsgemäßen Laserkatheters dargestellt. Der Laserkatheter ist von einer Ummantelung 1 umgeben, die den typischen Anforderungen für den Einsatz im medizinischen Bereich entspricht, wie beispielsweise leichte Sterilisierbarkeit, hohe Flexibilität und Materialverträglichkeit. Die äußere Ummantelung 1 umgibt die ringförmige Anordnung der Lichtleitfaser 2, die in dem in Figur 1 dargestellten Ausführungsbeispiel doppellagig, zu zwei konzentrischen Kreisen angeordnet sind. Dies geht ebenso aus der in Figur 1 dargestellten Draufsicht auf die Spitze des Laserkatheters hervor. In Verbindung mit einer inneren schlauchförmig ausgestalteten Ummantelung 3 wird ein Hohlkanal 4 umschlossen, der an seinem proximalen Ende mit einer Unterdruckquelle verbunden ist (in Figur 1 nicht dargestellt). An der Spitze des distalen Bereiches des erfindungsgemäßen Laserkatheters ist ein, den Außenumfang erweiterndes Element 5 vorgesehen, das in einer bevorzugten Ausführungsform einen atraumatischen ringformähnlichen Querschnitt aufweist, der in Richtung des distalen Endes eine geradlinig ausgearbeitete Stoßkante vorsieht. Die Dimensionierung des den Außenumfang erweiternde Element 5 muß dabei derart vorgenommen werden, daß der äußere Umfang des den Außenumfang des Laserkatheters erweiternden Elementes zumindest den Durchmesser des Gefäßkanales aufweist, durch den der Laserkatheter geführt werden soll. Somit ist gewährleistet, daß die Katheterspitze durch das umseitige Anliegen an der Innenfläche der Gefäßwand selbstführend zentral innerhalb des Gefäßkanals zentriert ist.

In Richtung des distalen Endes der Laserkatheterspitze ragen über die Ebene des den Außenumfang des Laserkatheter erweiternden Elementes 5 die ringförmigen Lichtleitfasern 2 heraus, die ihrerseits eine Halterung 6 umfassen, die proximalseitig einen Abschluß für die innere Ummantelung 3 darstellt, aber insbesondere eine Haltevorrichtung für den Abschlußdeckel 7 vorsieht, der den im inneren des Hohlkanals 4 vorherrschenden Unterdruck an die Oberseite des Abschlußdeckels 7 führt. Aus der in Figur 1 oberen Darstellung, einer Draufsicht auf die Laserkatheterspitze, ist eine vorteilhafte Anordnung von Perforationsbohrungen zu entnehmen, die auf konzentrischen Kreisen angeordnet sind. Ebenso ist aus der Draufsichtdarstellung die Umfangsfläche des den Außenumfang des Laserkatheters erweiternden Elementes 5 zu entnehmen.

Typische Abmaße des Laserkatheters sind wie folgt dimensioniert: Der Außendurchmesser der Ummantelung 1 des Laserkatheters beträgt 2,2 mm, wohingegen der Durchmesser am distalen Endbereiches durch das Element 5 auf 3 mm ansteigt. Die Lichtleitfasern 2 ragen distal typischerweise 1,5 mm aus dem Element 5 hervor und weisen einen Außendurchmesser von etwa 1,9 mm auf. Etwa 0,5 mm von der Lichtaustrittsfläche entfernt ist im Inneren der Lichtleitfasern 2 der Abschlußdeckel 7 eingefaßt, der in der Figur 1 dargestellten Form achtzehn Bohrungen mit jeweils einem Durchmesser von 0,2 mm aufweist. Die angegebenen Größen sind jedoch an die durch die Gefäßdimensionierung vorgegebenen Größenverhältnisse entsprechend anzupassen.

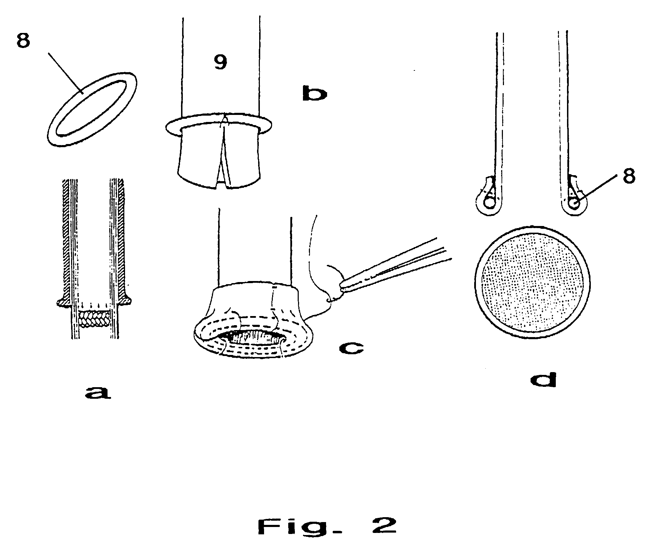

Für eine nähere Beschreibung der Funktionalität der erfindungsgemäßen Laserkatheterspitze ist in Figur 2 bis Figur 5 in schematischen Einzelbildsequenzen die Anwendungsweise der erfindungsgemäßen Vorrichtung zur Durchführung einer Bypass-Operation ohne Unterbrechung des Blutstromes innerhalb des blutführenden Gefäßes dargestellt.

Figur 2a zeigt die erfindungsgemäß ausgebildete Laserkatheterspitze über der ein ringförmiges Element 8 dargestellt ist, dessen Innendurchmesser größer ist als der Außendurchmesser der ringförmig angeordneten Lichtleitfasern und kleiner oder gleich bemessen ist als der Durchmesser des den Außenumfang des Laserkatheters erweiternden Elementes 5. Typischerweise beträgt der Innendurchmesser des beispielsweise aus einer körperverträglichen Platin-Iridium-Legierung hergestellten Rings 2,8 mm.

Für die Vorbereitung und Durchführung einer Bypass-Operation wird der Ring 8 auf einen Gefäßkanal 9 gestülpt, der aus einem anderen Körperteilbereich des zu behandelnden Patienten stammt, so daß die operative Anbindung des entnommenen Gefäßkanales, beispielsweise ein Stück Ader als Bypass an den blutführenden Gefäßkanal nicht durch körpereigene Abwehrreaktionen erschwert oder ganz behindert wird.

Gemäß Figur 2b wird sodann die entnommene Ader eingeschnitten und in der nach Figur 2c dargestellten Weise um den Ring 8 nach oben geklappt und mittels einer Naht um diesen befestigt. Auf diese Weise wird ein stabiler Gefäßkanalabschluß erhalten, der gemäß Figur 2d die Außenkontur des Ringes 9, in stabiler Form annimmt.

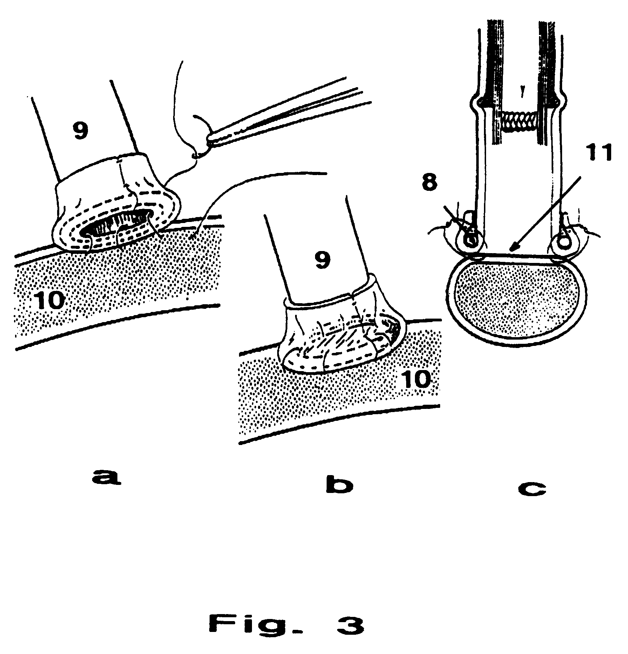

In einem weiteren Schritt wird der so vorbereitete Gefäßkanal 9 mit der Außenseite des behandelnden Gefäßkanales 10 durch eine ringförmig anzubringende Naht verbunden (Fig. 3a). Im Zustand gemäß Figur 3b sitzt der Bypass-Gefäßkanal 9 fest auf der Oberfläche des Gefäßkanals 10. Durch das Innere des Gefäßkanals 9 wird dann der erfindungsgemäße Laserkatheter in Richtung der zu durchtrennenden Gefäßwand 11 des Gefäßkanals 10 geführt. Wie aus Figur 3c hervorgeht, wird der Gefäßbereich 11 durch die Anbringung des Bypasses, bedingt durch den Ring 8 derart gestrafft, daß das distale Ende des Laserkatheters auf eine ebene Fläche aufsetzen kann. Dies führt zu dem Vorteil, daß während der Laserlichtapplikation die Gefäßwand 11 gleichmäßig im Kontaktbereich mit der Lichtaustrittsfläche mit Laserlicht beaufschlagt wird. Auf diese Weise wird für einen gleichmäßigen Materialabtrag an der Gefäßwand 11 gesorgt.

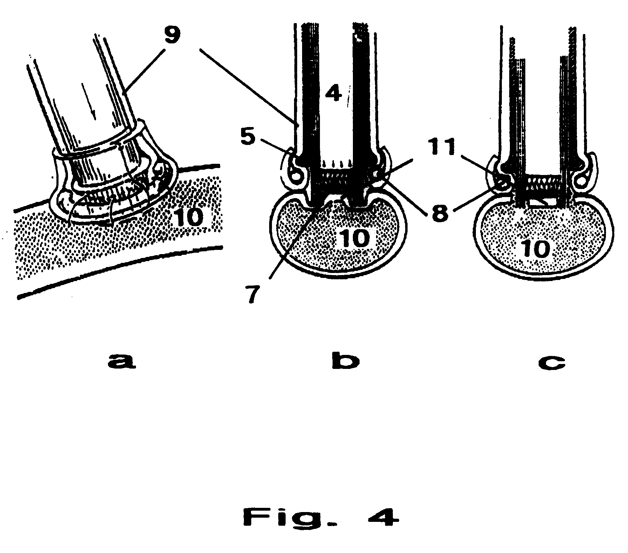

In Figur 4a ist eine perspektivische Darstellung der Verbindung beider Gefäßkanäle 9 und 10 sowie die Durchführung der Laserkatheterspitze durch den Gefäßkanal 9 dargestellt. Die Laserkatheterspitze wird wie aus Figur 4b zu entnehmen ist, zunächst solang durch den Gefäßkanal 9 geführt, bis die Lichtaustrittsfläche der distalen Laserkatehterspitze auf der abzutrennenden Gefäwand 11 aufliegt. Nun wird die Unterdruckquelle, die mit dem Hohlkanal 4 verbunden ist, aktiviert und sorgt dafür, daß das abzutrennende Gefäßwandstück 11 an die Oberfläche des Abschlußdeckels 7 gezogen wird.

Im weiteren wird die Laserlichtquelle aktiviert, die in dem vorgestellten Anwendungsfall bevorzugter Weise ein Excimer-Laser zur Erzeugung von ultravioletter Strahlung ist. Der Laser wird im Pulsbetrieb etwa 5 Sekunden lang mit einer Wiederholfrequenz von 40 Hz betrieben, so daß etwa 200 Pulse mit jeweils einer Energie von 15 mJ das Gewebe beaufschlagen. Die Laserkatheterspitze dringt sodann langsam in das Lumen des Gefäßkanals 10 ein bis die Stoßkante des den Außenumfang des Laserkatheters erweiternden Elementes 5 an der Gefäßwand anstößt, die durch den Ring 8 nach innen gedrückt wird. Das Gefäßwandstück 11 ist sodann gemäß Figur 4c von der übrigen Gefäßwand des Gefäßkanals 10 abgetrennt und haftet auf der Oberfläche des Abschlußdeckels 7 an.

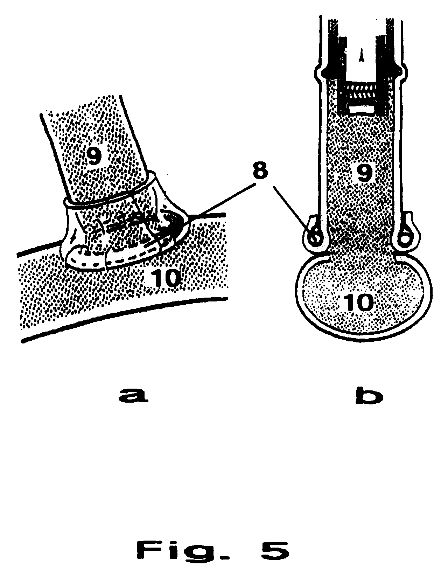

Gemäß den Figuren 5a und b kann nun das Blut auch über den Gefäßkanal 9 abfließen, nachdem das distale Ende des Laserkatheters samt abgetrennten Gefäßwandstück 11 aus den Bypass-Gefäßkanal 9 entnommen worden ist.

Der Platin-Iridium-Ring 8 verbleibt ständig an der Bypass-Verbindung zwischen den Gefäßkanälen 9 und 10.

Mit Hilfe der erfindungsgemäßen Vorrichtung ist es erstmals möglich, Bypass-Operationen durchzuführen, ohne den Blutstrom in den zu behandelnden Blutgefäßen zu unterbrechen. Derartige Unterbrechungen können insbesondere im Gehirnbereich zu großen Gefahren für den Patienten führen. Mit Hilfe der erfindungsgemäßen Laserkatheterspitze, die in Verbindung mit der erfindungsgemäßen ringförmigen Vorrichtung zu verwenden ist, können exakte Gefäßwanddurchbrüche erzeugt werden, ohne Gefäßwandreste in der Blutbahn zurückzulassen, die möglicherweise verengte Blutbahnstellen verschließen können. Ohne weitere Einschränkungen für etwaige Verwendungsmöglichkeiten der beschriebenen Katheterspitze im medizienischen Bereich ist die Anwendung der Vorrichtung bei jedlichen intrkorporalen Gefäßen denkbar, insbesondere auch für Bypass-Operationen der Herzkranzgefäße.

## Patentansprüche

1. Laserkatheter zur gezielten Applikation von Laserlicht an intrakorporalen Gefäßwänden, mit ringförmig angeordneten Lichtleitfasern (2), die einen inneren Hohlkanal (4) umschließen,
dadurch **gekennzeichnet,** daß am distalen Bereich des Laserkatheters ein den Außenumfang erweiterndes Element (5) vorgesehen ist, das über eine starre Form verfügt,
die in Strahlrichtung eine Stoßfläche als Anschlagvorrichtung aufweist, und daß die Lichtleitfasern (2) das den Außenumfang erweiternde Element (5) in Strahlrichtung überragen und als distale Laserkatheterspitze die Form einer ebenen, vollkreisringförmigen Lichtaustrittsfläche annehmen.

2. Laserkatheter nach Anspruch 1
dadurch **gekennzeichnet**, daß der Hohlkanal (4) am distalen Bereich einen perforierten Abschlußdeckel (7) aufweist, der geringfügig von der ebenen, ringförmigen Lichtaustrittsfläche der Lichtleitfasern (2) entgegen der Strahlrichtung beabstandet ist.

3. Laserkatheter nach Anspruch 2,
dadurch **gekennzeichnet**, daß der Abstand von Abschlußdeckel (7) und Lichtaustrittsfläche in Abhängigkeit von der zu durchtrennenden Gefäßwanddicke derart abhängt, daß der Abstand wenigstens der Gefäßwanddicke entspricht.

4. Laserkatheter nach Anspruch 2 oder 3,
dadurch **gekennzeichnet**, daß die Perforation des Abschlußdeckels (7) konzentrisch angeordnete Bohrungen aufweist.

5. Laserkatheter nach einem der Ansprüche 2 bis 4,
dadurch **gekennzeichne**t, daß der Abschlußdeckel (7) in einer Halterung (6) sitzt, die das den Außenumfang erweiternde Element (5) in Strahlrichtung überragt und zugleich von den Lichtleitfasern (2) umschlossen ist.

6. Laserkatheter nach einem der Ansprüche 1 bis 5,
dadurch **gekennzeichnet**, daß das am distalen Bereich des Laserkatheters vorgesehene, den Außenumfang erweiternde Element (5) während des Applikationsvorganges mit einem zweiten Element (8) in Wirkverbindung steht, das als Abstandshalter an einer Gefäßwand (11) angebracht ist.

7. Laserkatheter nach Anspruch 6,
dadurch **gekennzeichnet**, daß die Stoßfläche des den Außenumfang erweiternden Elements (5) mit einer äquivalent zu dieser ausgebildeten Stoßfläche des zweiten Elementes (8) in Wirkverbindung steht.

8. Laserkatheter nach Anspruch 6 oder 7,
dadurch **gekennzeichnet**, daß die Laserkatheterspitze während des Applikationsvorganges das zweite Element (8) durchragt, mit einer maximalen Durchdringungstiefe, die dem Abstand zwischen der Stoßfläche des den Außenumfang erweiternden Elements (5) und der Lichtaustrittsfläche des Laserkatheters entspricht.

9. Laserkatheter nach einem der Ansprüche 6 bis 8,
dadurch **gekennzeichnet**, daß die Durchdringungstiefe der Laserkatheterspitze derart bemessen ist, so daß eine saubere Durchtrennung der Gefäßwand (11) möglich ist ohne angrenzende oder gegenüberliegende Gefäßwandungen zu verletzten.

10. Laserkatheter nach einem der Ansprüche 6 bis 9,
dadurch **gekennzeichnet**, daß das zweite Element (8) ein Ring aus einer Platin-Iridium Legierung ist.

11. Laserkatheter nach einem der Ansprüche 1 bis 10,
dadurch **gekennzeichnet**, daß an dem Hohlkanal (4) eine Unterdruckquelle angeschlossen ist.

## Claims

1. Laser catheter for bypass surgery for selective laser light application on intracorporeal vascular walls, comprising optical fibres (2) in an annular arrangement, which enclose an internal cavity,,
**characterised** in that an element (5) is provided on the distal portion of the laser catheter, which extends the outer periphery and which has a rigid shape having a joint face as stop means in the beam direction, and that said optical fibres (2) project beyond said element (5) which extending the outer periphery, in the beam direction and take on the shape of a plane light emergence surface having a full circular shape, constituting the tip of the laser catheter.

2. Laser catheter according to Claim 1,
**characterised** in that the hollow channel (4) on the distal portion presents a perforated cover plate (7) which is slightly spaced from the plane annular light emergence surface of said optical fibres (2) in opposition to the beam direction.

3. Laser catheter according to Claim 2,
**characterised** in that the space between said cover plate (7) and the light emergence surface is dependent on the thickness of the vascular wall to be severed in such a way that the space corresponds at least to the thickness of the vascular wall.

4. Laser catheter according to Claim 2 or 3,
**characterised** in that the perforation in said cover plate (7) comprises concentrically disposed bores.

5. Laser catheter according to any of the Claims 2 to 4,
**characterised** in that said cover plate (7) is located in a mounting (6) which projects beyond said element (5) which extends the outer periphery, in the beam direction, and is enclosed at the same time by said optical fibres (2).

6. Laser catheter according to any of the Claims 1 to 5,
**characterised** in that said element (5) provided on the distal portion of the laser catheter and extending the outer periphery is operatively connected to a second element (8) during the application procedure, which second element is mounted as spacer on a vascular wall (11).

7. Laser catheter according to Claim 6,
**characterised** in that said joint face of said element (5) extending the outer periphery is operatively connected to a joint face of said second element (8), which presents an equivalent configuration.

8. Laser catheter according to Claim 6 or 7,
**characterised** in that the tip of the laser catheter projects through said second element (8) during the application procedure, at a maximum depth of penetration which corresponds to the spacing between the joint face of said element (5) extending the outer periphery and the light emergence surface of the laser catheter.

9. Laser catheter according to any of the Claims 6 to 8,
**characterised** in that the depth of penetration of the tip of the laser catheter is so dimensioned that an appropriate severance of said vascular wall (11) is possible without any lesion of the adjacent or opposite vascular walls.

10. Laser catheter according to any of the Claims 6 to 9,
**characterised** in that said second element (8) is a ring made of a platinum-iridium alloy.

11. Laser catheter according to any of the Claims 1 to 10,
**characterised** in that a vacuum source is connected to said hollow channel (4).

## Revendications

1. Cathéter laser pour une application ciblée de la lumière laser dans des parois vasculaires intracorporelles avec des fibres guide lumière (2) disposées en anneaux, et qui renferment un canal creux, (4) interne, caractérisé en ce que sur la zone distale du cathéter laser est prévu un élément (5) élargissant la périphérie extérieure, élément qui dispose d'une forme rigide, qui présente dans la direction des rayons une face d'appui en tant que dispositif de butée et en ce que les fibres du guide lumière (2) sont en surplomb sur l'élément (5) élargissant la périphérie extérieure dans la direction des rayons et comme pointe de cathéter laser distale prennent la forme d'une phase de sortie de lumière plane, entièrement circulaire.

2. Cathéter laser selon la revendication 1, caractérisé en ce que le canal creux (4) présente sur la zone distale un couvercle d'obturation perforé (7) qui est séparé de la phase de sortie de lumière plane annulaire des fibres de guide lumière (2) en opposition à la direction des rayons.

3. Cathéter laser selon la revendication 2, caractérisé en ce que la distance entre le couvercle d'obturation (7) et la phase de sortie de lumière est fonction de l'épaisseur de paroi vasculaire à séparer de sorte que la distance correspond au moins à l'épaisseur de paroi vasculaire.

4. Cathéter laser selon la revendication 2 ou 3, caractérisé en ce que la perforation du couvercle d'obturation (7) présente des alésages disposés concentriquement.

5. Cathéter laser selon l'une des revendications 2 à 4, caractérisé en ce que le couvercle d'obturation (7) repose dans un support (6) qui est en surplomb par rapport à l'élément (5) élargissant la périphérie extérieure dans le sens de rayonnement et en même temps renfermé par les fibres guide lumière (2).

6. Cathéter laser selon l'une des revendications 1 à 5, caractérisé en ce que l'élément (5) élargissant la périphérie extérieure, prévue sur la zone distale du cathéter laser est en liaison opérante pendant l'opération d'application avec un deuxième élément (8) qui est mis en place comme support écarteur sur une paroi vasculaire (11).

7. Cathéter laser selon la revendication 6, caractérisé en ce que la phase d'appui de l'élément (5) élargissant la périphérie extérieure est en liaison opérante avec une phase d'appui formée de façon équivalente du deuxième élément (8).

8. Cathéter laser selon la revendication 6 ou 7, caractérisé en ce que la pointe du cathéter laser traverse le deuxième élément (8) pendant l'opération d'application, avec une profondeur de pénétration maximum qui correspond à la distance entre la phase d'appui de l'élément (5) élargissant la périphérie extérieure et la phase de sortie de lumière du cathéter laser.

9. Cathéter laser selon l'une des revendications 6 à 8, caractérisé en ce que la profondeur de pénétration de la pointe du cathéter laser est dimensionnée de façon à permettre une séparation nette de la paroi vasculaire (11) sans léser les parois vasculaires contiguës ou opposées.

10. Cathéter laser selon l'une des revendications 6 à 9, caractérisé en ce que le deuxième élément (8) est un anneau constitué par un alliage platine-iridium.

11. Cathéter laser selon l'une des revendications s1 à 10, caractérisé en ce que sur le canal creux (4) est raccordée une source de dépression.
